(19) Europäisches Patentamt
European Patent Office
Office européen des brevets

(11) Publication number: 0 257 701 A1

# (12) EUROPEAN PATENT APPLICATION

(21) Application number: 87201548.2

(22) Date of filing: 14.08.87

(51) Int. Cl.[4]: C07D 471/04 , C07D 333/76 , C07D 307/91 , C07D 209/88 , C07D 491/048 , C07D 495/04 , C07H 17/00

(30) Priority: 15.08.86 NL 8602080

(43) Date of publication of application: 02.03.88 Bulletin 88/09

(84) Designated Contracting States: AT BE CH DE ES FR GB GR IT LI LU NL SE

(71) Applicant: Cedona Pharmaceuticals B.V.
Oudeweg 147
NL-2031 CC Haarlem(NL)

(72) Inventor: Langendoen, Albert
Langenweg 9c
NL-3235 CK Rockanje(NL)
Inventor: Koomen, Gerrit-Jan
Middelhof 20
NL-1851 BX Heiloo(NL)
Inventor: Pandit, Upendra Kumar
Stichtstraat 41
NL-1079 RC Amsterdam(NL)

(74) Representative: Smulders, Theodorus A.H.J. et al
Vereenigde Octrooibureaux Nieuwe Parklaan 107
NL-2587 BP 's-Gravenhage(NL)

## (54) A process for preparing hydroxyl derivatives of compounds containing a carbazole, dibenzofurane or dibenzothiophene group.

(57) Hydroxyl derivatives of compounds containing a carbazole, dibenzofurane or dibenzothiophene group, such as ellipticine, are prepared by selectively introducing an $R^3$-CO group, in which $R^3$ is hydrogen or alkyl, and then converting the $R^3$-CO group into a hydroxyl group.

EP 0 257 701 A1

**A process for preparing hydroxyl derivatives of compounds containing a carbazole, dibenzofurane or dibenzothiophene group.**

This invention generally relates to a process for preparing compounds containing the ring system according to formula 1:

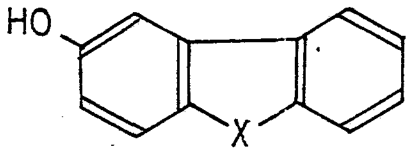

(1)

wherein X represents NR, O or S;
R represents H, alkyl, benzyl, phenyl, benzhydryl, $SO_2R^1$, $COR^1$, $COOR^1$;
$R^1$ represents alkyl, benzyl, phenyl, benzhydryl;
which compounds may contain substituents such as alkyl groups, halogen atoms, nitro groups, sulfonyl groups, ester groups, and sugar residues; and, if desired, may contain a ring system extended by one or more further rings, such as compounds containing the ring system according to formulae 2-5:

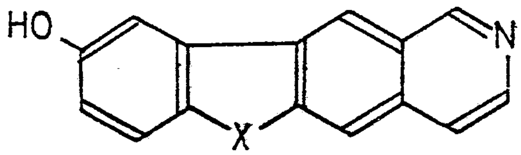

(2)

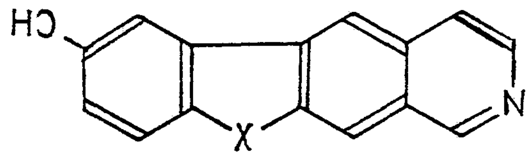

(3)

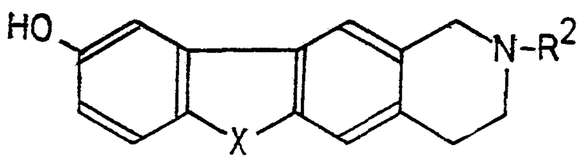

(4)

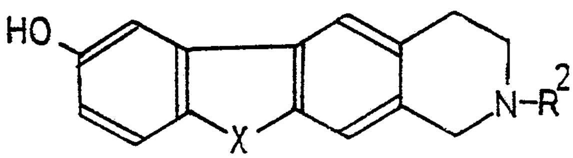

(5)

in which $R^2$ may have the same meanings as R, and, if desired, quaternized salts thereof. As used herein, quaternized salts means not only the salts of organic or inorganic acids, but also the reaction products with a compound R'-Y, in which Y represents a halogen atom and R' has the above meaning.

The invention relates more particularly to a process for preparing compounds having formula 8:

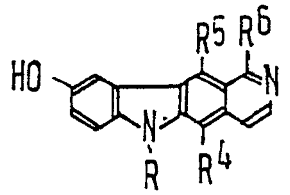

(8)

in which R represents H, alkyl, benzyl, phenyl, benzhydryl, $SO_2R^1$, $COR^1$, $COOR^1$;
$R^1$ represents alkyl, benzyl, phenyl, benzhydryl;
$R^4$ represents hydrogen, alkyl, benzyl, phenyl, benzhydryl, the group $CHR^{10}-(CHR^9)_n-A$;
$R^5$ represents hydrogen, alkyl, benzyl, phenyl, benzhydryl, the group $CHR^{10}-(CHR^9)_n-A$;
$R^6$ represents hydrogen or, if $R^5$ represents hydrogen, may alternatively represent alkyl, benzyl, phenyl, benzhydryl, the group $CHR^{10}-(CHR^9)_n-A$; and wherein n is 0 or 1; $R^8$ represents hydrogen, alkyl or aryl; $R^{10}$ represents hydrogen, alkyl, aryl or a possibly derivatized hydroxymethyl group; A represents a possibly substituted hydroxyl or amino group, such as a sugar group or an $N(CH_2-CH_2Cl)_2$ group, and in which the various groups may carry further substituents, such as alkyl groups,halogen atoms, nitro groups, sulfonyl groups, ester groups, and sugar residues.

Among these compounds, it is especially 9-hydroxyellipticine (formula 8, $R = R^6 = H$, $R^4 = R^5 = $ methyl) (9-HE) and its derivatives which are of importance by reason of their anti-tumor activity. It has been found that the 9-hydroxy derivatives generally have a stronger anti-tumor activity than have the corresponding non-substituted compounds. Thus, for example, 9-HE is forty times more active against leukaemia thal ellipticine itself.

9-HE is a known compound which can be prepared, for example, starting from 5-hydroxy-indole. This process has the disadvantage, however, that the starting material, 5-hydroxy-indole, is difficult to prepare and hence expensive. The next stages in the synthesis are cumbersome and have low yields, so that this route of synthesis as a whole is unattractive.

Gouyette et al. further describe a process for the preparation of 9-HE from 9-methoxy-ellipticine (9-ME) by heating 9-ME in the presence of pyridinium chloride (Eur. J. Med. Chem-Chim. Ther., 1980, 15, 503).

9-methoxy-ellipticine itself, however, is prepared in a process which is also cumbersome, starting from 5-hydroxy-indole, so that it offers no advantages over the above process.

It may also be endeavoured to use the natural product ellipticine as the starting product, and to prepare 9-HE from this by a hydroxylation reaction. Generally speaking, however, it is difficult to introduce a substituent into a polycyclic system like the present one in a somewhat selective manner, that is to say, without obtaining mixtures of isomers. In C.R. Acad. Sc. Paris 281 (1975), 623-626, a process is described for the preparation of 9-HE by subjecting ellipticine successively to bromination, amination, diazotization, and hydrolyzing the diazonium salt. The efficiency of this extremely laborious multi-stage synthesis is approximately 30%.

There is accordingly a need for a process for the preparation of hydroxyl derivatives of ellipticine and similar compounds containing the ring system

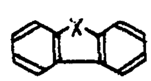

and which process does not have the above disadvantages.

Surprisingly it has now been found that such a process is provided by the process according to the present invention, which is characterized by converting a corresponding compound containing the ring system of formula 6

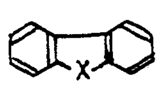

(6)

into a compound containing the ring system of formula 7

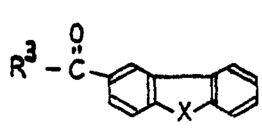

(7)

in which $R^3$ represents hydrogen or alkyl;
converting the $R^3$-CO group into an OH group, and if desired providing the resulting compound with certain substituents and/or, if desired, converting it into a quaternized salt.

Preferably, a compound of formula 8 is prepared by converting a corresponding compound having formula 9:

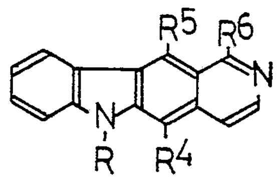

(9)

in which R represents H, alkyl, benzyl, phenyl, benzhydryl, $SO_2R^1$, $COR^1$, $COOR^1$;
$R^1$ represents alkyl, benzyl, phenyl, benzhydryl;
$R^4$ represents hydrogen, alkyl, benzyl, phenyl, benzhydryl;
$R^5$ represents hydrogen, alkyl, benzyl, phenyl, benzhydryl;
$R^6$ represents hydrogen, or, if $R^5$ represents hydrogen, may also represent alkyl, benzyl, phenyl, benzhydryl;
and in which the various groups may carry further substituents, such as alkyl groups, halogen atoms, nitro groups, sulfonyl groups, ester groups;
into a compound having formula 10:

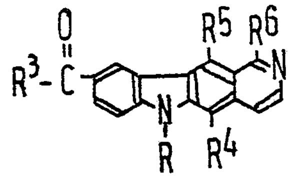

(10)

in which R, $R^4$, $R^5$ and $R^6$ have the meanings specified above, and $R^3$ represents hydrogen or alkyl;
converting this compound having formula 10 into a compound having formula 8, in which R, $R^4$, $R^5$ and $R^6$ have the above meanings;
if desired coupling a sugar residue to one or more of the side groups present; and/or converting one or more of the side groups present into another side group; and/or forming a quaternized salt.

Particularly preferably, there is prepared a 9-hydroxy-ellipticine derivative having formula 11

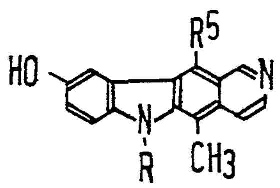

(11)

in which R represents hydrogen or methyl;
$R^5$ represents methyl or the group $CHR^{10}-(CHR^9)_n-A$;
n is 0 or 1; $R^9$ represents hydrogen, alkyl or aryl;
$R^{10}$ represents hydrogen, alkyl, aryl or a possibly derivatized hydroxymethyl group; A represents a possibly substituted hydroxyl or amino group, such as a sugar group or an $N(CH_2-CH_2Cl)_2$ group by converting a corresponding compound having formula 12:

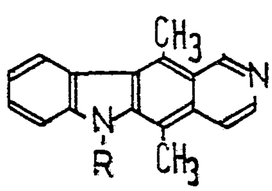

(12)

in which R represents hydrogen, alkyl, benzyl, phenyl, benzhydryl, $SO_2R^1$, $COR^1$, $COOR^1$, and $R^1$ represents alkyl, benzyl, phenyl, benzhydryl;
into a compound having formula 13:

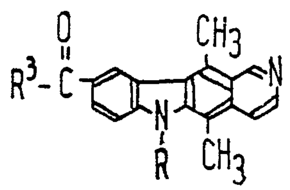

(13)

in which R has the above meanings and $R^3$ represents hydrogen or alkyl;
converting this compound having formula 13 into a compound having formula 11, in which R has the above meanings and $R^5$ represents methyl or $CHR^{10}$-$(CHR^9)_n$-A, wherein n is 0 or 1; $R^9$ represents hydrogen, alkyl or aryl; $R^{10}$ represents hydrogen, alkyl, aryl or a possibly derivatized hydroxymethyl group; A represents a possibly substituted hydroxyl or amino group, such as a sugar group or an $N(CH_2$-$CH_2Cl)_2$ group;
if desired removing group R if it does not represent hydrogen, or, if it is not a methyl group, converting it into a methyl group.

The first stage of the reaction is an aromatic substitution, in which an $R^3$-CO group is introduced selectively. In this formula, $R^3$ represents a hydrogen atom or an alkyl group. This can be effected using the Friedel-Crafts acylation, in which an acid chloride $R^3$-COX, in which X represents a halogen atom and $R^3$ represents an alkyl group, or a carboxylic anhydride is reacted with an aromatic compound in the presence of a catalyst. Suitable catalysts are Lewis acids, such as $AlCl_3$, $FeCl_3$, $SnCl_4$, etc.

It is also possible to use the Vilsmeyer reaction to introduce a formyl group.

For this purpose the conversion is carried out with a compound having the formula $HCON(R^8)_2$, in which $R^8$ represents an alkyl group, in the presence of $POCl_3$. The preferred solvent here is dimethyl formamide. Preferably, however, an equivalent of the Friedel Crafts acylation is used, for example, the reaction with $Hal_2CH$-O-$R^7$, in which hal represents a halogen, such as Cl or Br and $R^7$ represents an alkyl, benzyl or benzhydryl group. The compound dichloromethylmethyl ether has been found to be very suitable.

The reaction proceeds under the influence of any Friedel Crafts catalyst, but preferably in the presence of $AlCl_3$. Any solvent suitable for carrying out a Friedel Crafts acylation, such as anhydrous THF, ether, nitrobenzene, $CH_2Cl_2$, etc., can be used. It is of importance that the reaction is carried out under dry conditions, preferably under an atmosphere of a dry inert gas, such as nitrogen.

The reaction is carried out at a temperature of -30 to +30°, preferably a temperature of about 0°C.

In this first stage, the selectivity of the acylation is surprising, because aromatic substitution reactions often produce mixtures of products. Thus applicants have found that the nitration of ellipticine leads to a mixture of isomers.

The product of the first reaction stage is subsequently processed in the usual manner, such as, for example, extraction with an organic solvent, washing the solvent layer with aqueous solutions, evaporating the solvent and purifying, e.g., over a silica gel column.

The second reaction stage is the conversion of the $R^3$-CO or H-CO compound formed in the first stage. this conversion is carried out as a Baeyer-Villiger re-arrangement, in which an aromatic aldehyde or ketone is reacted with a hydroperoxide, such as a peracid or hydrogen peroxide in acid medium to produce a phenol-type compound.

In the reaction, an organic peracid in an organic solvent can be used. Preferably, however, the reaction is carried out using 30% hydrogen peroxide and an inorganic acid, as in that case, there is virtually no oxidation of any unprotected nitrogen atoms that may be present in the molecule.

If desirable for the solubility of reactants of the product, a water-miscible organic solvent, such as methanol, may be added, if desired.

The reaction temperature is not particularly critical; preferably, the reaction is carried out under reflux conditions. After completion of the reaction, the reaction product is refined and purified by known methods.

Surprisingly, there are hardly any side reactions in this second stage, such for example as the oxidation of methyl groups or the oxidation of the aldehyde to form a carboxylic acid.

After the introduction of an OH group into the molecule in the above manner, other substituents may be introduced or O-or N-derivatives may be prepared, e.g. sugar residues may be attached to one or more of the side groups as desired. Also, one or more of the side groups may be converted into a different group, or a quaternized derivative can be prepared.

Thus, for example, for better solubility, a sugar group may be attached to the molecule by converting one of the methyl groups into a $CH_2$-$(CHR^9)_n$-OH group, in which n is 0 or 1, and $R^9$ represents hydrogen, alkyl or aryl, and coupling a sugar residue to this in a manner known per se.

For this purpose, for example, the hydroxylated compound, preferably a compound having formula 15, if necessary after protecting the hydroxyl group with a base, is converted into it anion, which is next reacted with a compound having formula $R^9$-CHO, in which $R^9$ is hydrogen, alkyl, or aryl, such as phenyl. A suitable compound is, for example, paraformaldehyde.

Preferably, a compound is formed having the formula

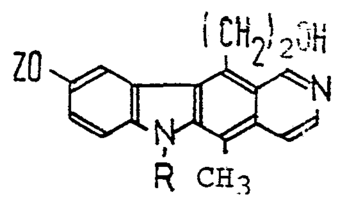

(16A)

in which Z represents hydrogen or a protective group. Thereafter, the compound is reacted with a sugar, possibly activated in one position and for the rest protected. Suitable sugars are all known sugar residues, both mono-and oligosaccharides, e.g., glucose, galactose, maltose, etc. Thereafter the protective groups are removed.

Finally, the compounds formed may be converted into their quaternized salts, which makes them less sensitive to oxidation and better soluble. These salts are prepared in known manner.

The invention is illustrated in and by the following example, which is given by way of illustration only, and must not be construed as limiting the invention in any way.

Example

A. Formylation of 6-methylellipticine.

6 g aluminium chloride (45 mmoles) was added to 225 ml dry dichloromethane under nitrogen, and the suspension was stirred at room temperature for 10 minutes. To the suspension, 5.85 g 6-methylellipticine (22.5 mmoles) was supplied, the mixture was stirred at room temperature for 10 minutes, and then cooled to 0°C. Subsequently, 4 ml dichloromethylmethylether (45 mmoles) in 80 ml dry dichloromethane was added dropwise to the aluminum chloride suspension over 1.5 hours, and the mixture was stirred at 0°C for another 1.5 hours.

Thereafter the reaction mixture was poured into a solvent mixture of water (1.1 l) and chloroform (600 ml) and to this mixture $Na_2CO_3$ was added until a pH of about 8 was reached. The organic layer was separated and the water layer was twice extracted with chloroform.

The combined chloroform layers were washed with 200 ml saturated $Na_2CO_3$ solution and 200 ml saturated NaCl solution. After drying the chloroform solution on anhydrous $Na_2SO_4$ and evaporating the solvent, the product was obtained as a foam. This was stirred with 100 ml ethylacetate to give a crystalline product, which was separated by filtration. From the filtrate, another 0.6 g material was obtained. Total yield was 5.95 g (91% of theory), the melting point was 215°C. Further purification by means of column chromatography (silica gel, eluent: $CH_3OH/CH_2Cl_2$ 4:100) gave the pure product having a melting point of 223-226°C. Analysis gave the following results:

IR ($CHCl_3$: 1677, 1590 $cm^{-1}$. PMR ($CDCl_3$): $\delta$ 10.07 (s, 1H, -CHO), 9.64 (s, 1H, H-1), 8.66 (d, 1H, $J_{8,10}$ = 1.2, H-10), 8.52 (d, 1H $J_{3,4}$=5.9, H-3), 8.03 (dd, 1H, $j_{8,10}$ = 1.2, $J_{7,8}$ = 8.5, H-8), 7.85 (d, 1H, $J_{3,4}$ = 6.2, H-4), 7.37 (d, 1H, $J_{7,8}$ = 8.5, H-7), 4.06 (s, 3H, N-Me), 3.14, 2.96 (2xs, 6H, 5-Me + 11-Me).

B. Formation of 6-methyl-9-hydroxyellipticine.

4.23 g 6-methyl-9-formylellipticine (15 mmoles) was suspended in a mixture of 150 ml water and 150 ml methanol. To this mixture, 2.25 ml 95% $H_2SO_4$, followed by 3.75 ml 35% hydrogen peroxide solution were added with stirring. The resulting mixture was then refluxed for 22 hours, an additional portion of hydrogen peroxide was added (2 ml) and after cooling the mixture was poured into 200 ml water.

The red precipitate which separated in this stage was stirred with 200 ml $CHCl_3$ for 0.5 hour, whereafter 100 ml methanol was added and - dropwise -a solution of 90 g Na Ac. $3H_2O$ in 100 ml water. 1 l $CHCl_3$, 100 ml methanol and 500 ml water were added, the mixture was shaken, and the organic layer was separated, washed with 200 ml saturated NaCl solution and dried over $Na_2SO_4$. The solvent was removed by evaporation; 100 ml ethyl acetate was added to the residue, and the mixture was stirred, whereafter 3.34 g product was obtained. The filtrate produced another 0.51 g of material. The total yield was 3.85 g (83% of theory), with the melting point being > 350°C.

Analysis gave the following results:

IR (KBr): 1590, 1390, 1475 $cm^{-1}$. PMR (DMSO-$d_6$): 9.76 (s, 1H, h-1), 9.49 (s, 1H, OH), 8.39 (d,1H, $J_{3,4}$ = 6.4, H-3) 8.25 (d, 1H, $J_{3,4}$= 6.4, H-4) 7.80 (d, 1H, $J_{8,10}$ = 2, H-10), 7.52 (d, 1H, $J_{7,8}$ = 8,8, H-7), 7.6 (dd, 1H, $J_{7,8}$ = 8,8, $J_{8,10}$ = 2, H-8), 4.11 (s, 3H, $NCH_3$), 3.14 (s, 3H, 11-$CH_3$), 3.00 (s, 3H. 5-$CH_3$).

## Claims

1. A process for preparing compounds containing the ring system according to formula 1:

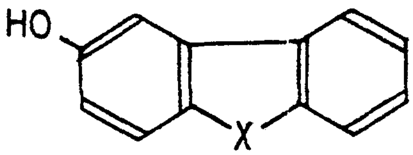

(1)

wherein X represents NR, O or S;
R represents H, alkyl, benzyl, phenyl, benzhydryl, $SO_2R^1$, $COR^1$, $COOR^1$;
$R^1$ represents alkyl, benzyl, phenyl, benzhydryl;
which compounds may contain substituents such as alkyl groups, halogen atoms, nitro groups, sulfonyl groups, ester groups, and sugar residues; and, if desired, may contain a ring system extended by one or more further rings, such as compounds containing the ring system according to formulae 2-5:

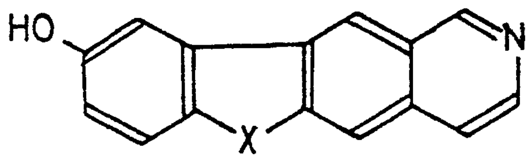

(2)

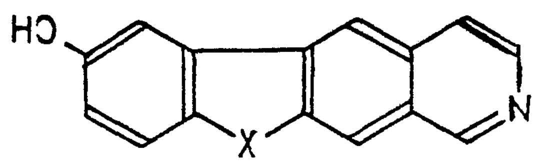

(3)

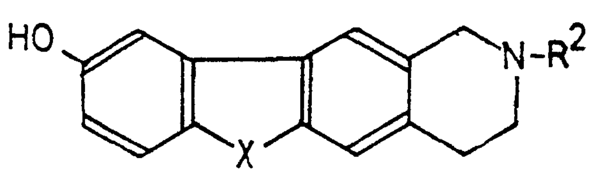

(4)

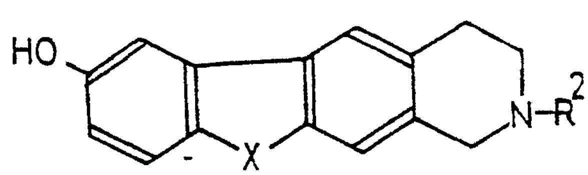

(5)

in which $R_2$ may have the same meanings as R, and, if desired, quaternized salts thereof, which comprises converting a corresponding compound containing the ring system of formula 6

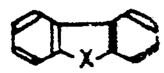

(6)

into a compound containing the ring system of formula 7

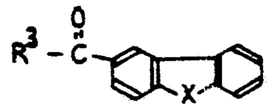

(7)

in which $R^3$ represents hydrogen or alkyl;
converting the $R^3$-CO group into an OH group, and if desired providing the resulting compound with certain substituents and/or, if desired, converting it into a quaternized salt.

2. A process as claimed in claim 1, preparing a compound having formula 8:

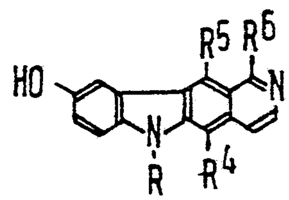

(8)

in which R represents H, alkyl, benzyl, phenyl, benzhydryl, $SO_2R^1$, $COR^1$, $COOR^1$;
$R^1$ represents alkyl, benzyl, phenyl, benzhydryl;
$R^4$ represents hydrogen, alkyl, benzyl, phenyl, benzhydryl, the group $CHR^{10}$-$(CHR^9)_n$-A;
$R^5$ represents hydrogen, alkyl, benzyl, phenyl, benzhydryl, the group $CHR^{10}$-$(CHR^9)_n$-A;
$R^6$ represents hydrogen or, if $R^5$ represents hydrogen, may alternatively represent alkyl, benzyl, phenyl, benzhydryl, the group $CHR^{10}$-$(CHR^9)_n$-A; and wherein n is 0 or 1; $R^9$ represents hydrogen, alkyl or aryl; $R^{10}$ represents hydrogen, alkyl, aryl or a possibly derivatized hydroxymethyl group; A represents a possibly substituted hydroxyl or amino group, such as a sugar group or an $N(CH_2$-$CH_2Cl)_2$ group, and in which the various groups may carry further substituents, such as alkyl groups, halogen atoms, nitro groups, sulfonyl groups, ester groups, and sugar residues, by converting a corresponding compound having formula 9:

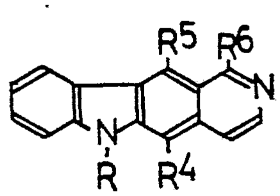

(9)

in which R represents H, alkyl, benzyl, phenyl, benzhydryl, $SO_2R^1$, $COR^1$, $COOR^1$;
$R^1$ represents alkyl, benzyl, benzyl, phenyl, benzhydryl;
$R^4$ represents hydrogen, alkyl, benzyl, phenyl, benzhydryl;
$R^5$ represents hydrogen, alkyl, benzyl, phenyl, benzhydryl;
$R^6$ represents hydrogen, or, if $R^5$ represents hydrogen, may also represent alkyl, benzyl, phenyl, benzhydryl;
and in which the various groups may carry further substituents, such as alkyl groups, halogen atoms, nitro groups, sulfonyl groups, ester groups;
into a compound having formula 10:

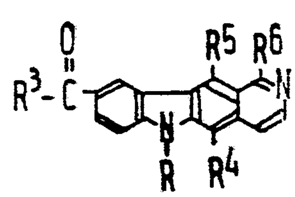

(10)

in which R, $R^4$, $R^5$ and $R^6$ have the meanings specified above, and $R^3$ represents hydrogen or alkyl; converting this compound having formula 10 into a compound having formula 8, in which R, $R^4$, $R^5$ and $R^6$ have the above meanings;
if desired coupling a sugar residue to one or more of the side groups present; and/or converting one or more of the side groups present into another side group; and/or forming a quaternized salt.

3. A process as claimed in claim 1 for preparing an ellipticine derivative having formula 11

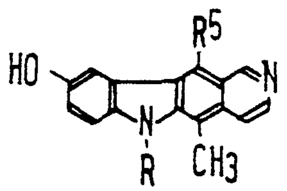

(11)

in which R represents hydrogen or methyl;
$R^5$ represents methyl or the group $CHR^{10}$-$(CHR^9)_n$ -A;
n is 0 or 1; $R^9$ represents hydrogen, alkyl or aryl;
$R^{10}$ represents hydrogen, alkyl, aryl or a possibly derivatized hydroxymethyl group; A represents a possibly substituted hydroxyl or amino group, such as sugar group or an $N(CH_2$-$CH_2Cl)_2$ group by converting a corresponding compound having formula 12:

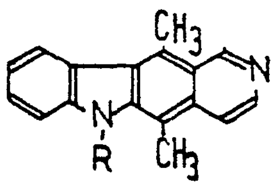

(12)

in which R represents hydrogen, alkyl, benzyl, phenyl, benzhydryl, $SO_2R^1$, $COR^1$, $COOR^1$, and $R^1$ represents alkyl, benzyl, phenyl, benzhydryl;
into a compound having formula 13:

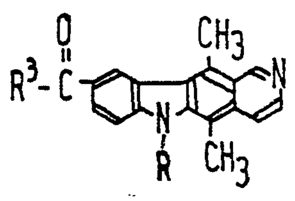

(13)

in which R has the above meanings and $R^3$ represents hydrogen or alkyl;
converting this compound having formula 13 into a compound having formula 11, in which R has the above meanings and $R^5$ represents methyl or $CHR^{10}$-(CHR)-A, wherein n is 0 or 1; $R^9$ represents hydrogen, alkyl or aryl; $R^{10}$ represents hydrogen, alkyl, aryl or a possibly derivatized hydroxymethyl group; A represents a possible substituted hydroxyl or amino group, such as a sugar group or an $N(CH_2$-$CH_2Cl)_2)$ group;
if desired removing group R if it does not represent hydrogen, or, if it is not a methyl group, converting it into a methyl group.

4. A process as claimed in any of claims 1-3, in which the first reaction stage comprises the introduction of a formyl group ($R^3$ = hydrogen).

5. A process as claimed in claim 4, wherein the formyl group is introduced by means of a Friedel and Crafts formylation reaction and a subsequent hydrolysis stage.

6. A process as claimed in claim 5, which comprises using as the formylation agent a compound having formula 14:

$Hal_2CH$-O-$R^7$ (14)

in which Hal represents a halogen, such as chlorine or bromine, and $R^7$ represents alkyl, benzyl or benzhydryl.

7. A process as claimed in claim 6, in which the formylation agent used is $Cl_2CH-O-CH_3$.

8. A process as claimed in any of claims 5-7, wherein the Friedel and Crafts catalyst used is $AlCl_3$.

9. A process as claimed in any of claims 5-8, in which the formylation reaction is carried out in an organic solvent while excluding water and at a temperature of between -30° and +30°C.

10. A process as claimed in claim 9, in which the reaction is carried out in dichloromethane at a temperature of 0-25°C.

11. A process as claimed in claim 4, wherein the formyl group is introduced by means of a Vilsmeyer reaction.

12. A process as claimed in claim 11, in which the conversion is carried out with a compound having the formula $HCON(R^8)_2$, in which $R^8$ represents alkyl, in the presence of $POC1_3$.

13. A process as claimed in claim 11 or 12, in which dimethylformamide is used as the solvent.

14. A process as claimed in any of claims 1-13, in which the second reaction stage is a Baeyer-Villiger re-arrangement.

15. A process as claimed in claim 14, in which the Baeyer-Villiger re-arrangement is carried out with a hydroperoxide, such as hydrogen peroxide or a percarboxylic acid, in an acid medium.

16. A process as claimed in claim 14 or 15, in which the Baeyer-Villiger re-arrangement is carried out with $H_2O_2$ in an aqueous, acidic medium.

17. A process as claimed in any of claims 14-16, in which the Baeyer-Villiger re-arrangement is carried out at a temperature between 0°C and the boiling point of the reaction mixture.

18. A process as claimed in any of claims 1-17, in which a sugar residue is attached to the hydroxylated compound by first converting a methyl substituent that is present into a $CH_2-(CHR^9)_n-OH$ group, in which n = 0 or 1 and $R^9$ represents hydrogen, alkyl or aryl, and coupling a sugar residue thereto in a manner known per se.

19. A process as claimed in claim 18, in which a compound having formula 15:

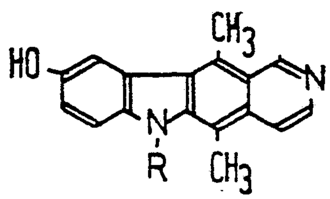

(15)

in which R represents hydrogen or methyl, is converted by means of a base and formaldehyde into a compound having formula 16:

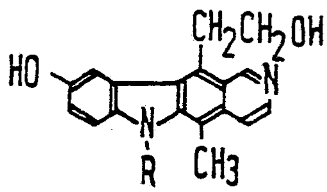

(16)

and that compound is reacted with a sugar, such as glucose, galactose, maltose, and the like, in which, if desired, one or more hydroxyl groups are activated or protected.

20. A process as claimed in any of claims 1-19, in which a compound having formula 15 is quaternized to produce a compound having formula 17:

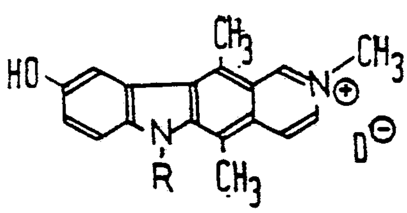

(17)

in which R represents hydrogen or methyl and $D^-$ represents an anion, such as acetate, iodide, and the like.

European Patent Office

# EUROPEAN SEARCH REPORT

Application Number

EP 87 20 1548

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF MEDICINAL CHEMISTRY, vol. 20, no. 3, 1977, pages 364-371; W.L. ALBRECHT et al.: "Bis-basic-substituted polycyclic aromatic compounds. A new class of antiviral agents.1-3 8. Bis-basic derivatives of carbazole, dibenzofuran, and dibenzothiophene" * Page 364, column 2, lines 12-14; page 365, scheemes I,II; column 1, lines 1-3,15-18; page 370, column 2, lines 18-27,46-54 * | 1 | C 07 D 471/04<br>C 07 D 333/76<br>C 07 D 307/91<br>C 07 D 209/88<br>C 07 D 491/048<br>C 07 D 495/04<br>C 07 H 17/00 |
| A | JOURNAL OF THE CHEMICAL SOCIETY, Perkin Transactions I, 1974, pages 1353-1354, London, GB; I.M. GODFREY et al.: "Preparation of methoxyphenols by Baeyer-Villiger oxidation of methoxy,benzaldehydes" * Page 1353, column 1 * | 1 | |
| | | | **TECHNICAL FIELDS SEARCHED (Int. Cl.4)** |
| | | | C 07 D 471/00 |

The present search report has been drawn up for all claims

| Place of search | Date of completion of the search | Examiner |
|---|---|---|
| THE HAGUE | 16-11-1987 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)